Europäisches Patentamt

European Patent Office

Office européen des brevets

(11) Veröffentlichungsnummer: **0 126 197**
**B1**

## (12) EUROPÄISCHE PATENTSCHRIFT

(45) Veröffentlichungstag der Patentschrift :
06.05.87

(51) Int. Cl.⁴ : **A 61 M 5/14**

(21) Anmeldenummer : 83710055.1

(22) Anmeldetag : 24.08.83

(54) Dosiervorrichtung zur Einstellung einer konstanten Durchflussmenge für Blut, Blutersatzlösungen und ähnliche Flüssigkeiten.

(30) Priorität : 19.04.83 DE 8311508 U

(43) Veröffentlichungstag der Anmeldung :
28.11.84 Patentblatt 84/48

(45) Bekanntmachung des Hinweises auf die Patenterteilung : 06.05.87 Patentblatt 87/19

(84) Benannte Vertragsstaaten :
AT CH DE FR GB LI SE

(56) Entgegenhaltungen :
DE-A- 3 107 413
DE-B- 2 147 572
GB-A- 1 482 052

(73) Patentinhaber : Infusionstechnik Clinico GmbH & Co.
An der Haune 10
D-6430 Bad Hersfeld (DE)

(72) Erfinder : Heinzerling, Christian Walter
Sonnenhang 4
D-3509 Malsfeld-Beiseförth (DE)

(74) Vertreter : Patentanwälte Dipl.-Ing. Rudolf Bibrach
Dipl.-Ing. Elmar Rehberg
Pütterweg 6 Postfach 738
D-3400 Göttingen (DE)

**Beschreibung**

Die Erfindung bezieht sich auf eine Dosiervorrichtung mit den im ersten Teil des Anspruchs 1 angegebenen Merkmalen. Solche Dosiervorrichtungen werden insbesondere in der medizinischen Technik angewendet. Dabei kommt es darauf an, eine konstante Durchflußmenge hinsichtlich der erwähnten Flüssigkeit einzustellen, wobei die Menge z. B. in Tropfenform sehr klein sein kann, individuell eingestellt werden muß und die jeweils eingestellte Menge über die Zeit, also über den gesamten Infusions- und Transfusionszeitraum konstant bleiben soll.

Eine Dosiervorrichtung der eingangs beschriebenen Art ist aus der DE-B-2 147 572 bekannt. In einem rohrförmigen Hauptkörper ist ein als Verschlußkörper dienender beweglicher Teil axial verschieblich gelagert, der im Bereich zweier versetzt angeordneter Anschlußstutzen mit einer Einschnürung und einer sich in axialer Richtung erstreckenden Kerbe auf dem Umfang versehen ist. Die Kerbe arbeitet mit einem der beiden Anschlußstutzen zusammen, wobei dessen Durchbrechung mit der Kerbe von dem völligen Abschluß bis zur völligen Freigabe in Relativlage gebracht wird. Die Kerbe besitzt etwa dreieckigen Querschnitt und läuft in axialer Richtung mit sich änderndem Querschnitt an dem beweglichen Teil aus. Zwischen der Verdickung des beweglichen Teils und der Einschnürung ist eine umlaufende Kante vorgesehen, die jedoch keine Ventilfunktion erfüllt. Der bewegliche Teil gestattet es, durch ein Verdrehen den Durchsatz in gewissen Grenzen noch zu ändern.

Der Erfindung liegt die Aufgabe zugrunde, eine Dosiervorrichtung der eingangs beschriebenen Art derart weiterzubilden, daß sich eine konstante Durchflußmenge mit vergleichsweise größerer Genauigkeit einstellen läßt, so daß auch bei sehr kleiner Durchflußmenge beispielsweise eine konstante Tropfenzahl über den gesamten Übertragungszeitraum erzielbar bzw. einstellbar ist.

Erfindungsgemäß wird dies dadurch erreicht, daß der bewegliche Teil an dem Hauptkörper zum Zwecke der Ein- bzw. Verstellung der Durchflußmenge drehbar gelagert ist, und daß an einem in die Bohrung einragenden Fortsatz des beweglichen Teils stirnseitig eine Schließkante vorgesehen ist, die je nach Drehung des Teils die eine Durchbrechung mehr oder weniger verschließt. Damit wird das Blut vorteilhaft schonend behandelt, indem nur eine einzige Durchbrechung mehr oder weniger weit geöffnet wird, um die konstante Durchflußmenge einzustellen. Eine zusätzliche Einschnürung oder Teilung der Strömung findet nicht statt. Die Ventileinstellung durch Drehung arbeitet sehr feinfühlig und zuverlässig.

Der bewegliche Teil kann an dem Hauptkörper über eine umlaufende Rippe/Nut-Verbindung drehbar gelagert sein, während die Schließkante als schiefe Ebene ausgebildet ist. Durch die Anwendung der umlaufenden Rippe/Nut-Verbindung ist der bewegliche Teil lediglich drehbar, nicht aber axial verschieblich gelagert. Demzufolge ist es erforderlich, den Fortsatz des beweglichen Teils in einer schiefen Ebene enden zu lassen, die dann je nach Drehwinkel des beweglichen Teils relativ zum Hauptkörper die eine Durchbrechung mehr oder weniger verschließt bzw. freigibt.

Eine andere Ausführungsform besteht darin, daß der bewegliche Teil an dem Hauptkörper über eine Gewindeverbindung geführt ist, und daß die Schließkante rechtwinklig umlaufend zu der Achse des beweglichen Teils vorgesehen ist. Durch die Anwendung der Gewindeverbindung ist sichergestellt, daß der bewegliche Teil relativ zum Hauptkörper nicht nur gedreht, sondern dabei zwangsläufig auch axial versetzt wird. Auf diese Art und Weise deckt die Schließkante und die anschließende Wandung des Fortsatzes die Durchbrechung mehr oder weniger ab, so daß auch damit eine genaue und verläßliche Einstellung der Tropfenzahl über den gewünschten Zeitraum möglich ist.

Der Hauptkörper und der bewegliche Teil können zweckmäßig als Kunststoffspritzteile ausgebildet sein. Sie besitzen damit eine feste Gestalt und lassen sich einfach herstellen. Zwischen dem beweglichen Teil und dem Hauptkörper kann eine Dichtung angeordnet sein. Dies ist durch eine entsprechende Passung der Teile oder auch durch den Einsatz einer Ringdichtung aus elastomerem Material möglich.

Die Erfindung wird anhand zweier Ausführungsbeispiele weiter beschrieben. Es zeigen :

Figur 1 einen Vertikalschnitt durch die Dosiervorrichtung in einer ersten Ausführungsform,

Figur 2 einen Schnitt gemäß der Linie II-II in Figur 1 und

Figur 3 eine ähnliche Schnittdarstellung wie Figur 1, jedoch bei einer anderen Ausführungsform.

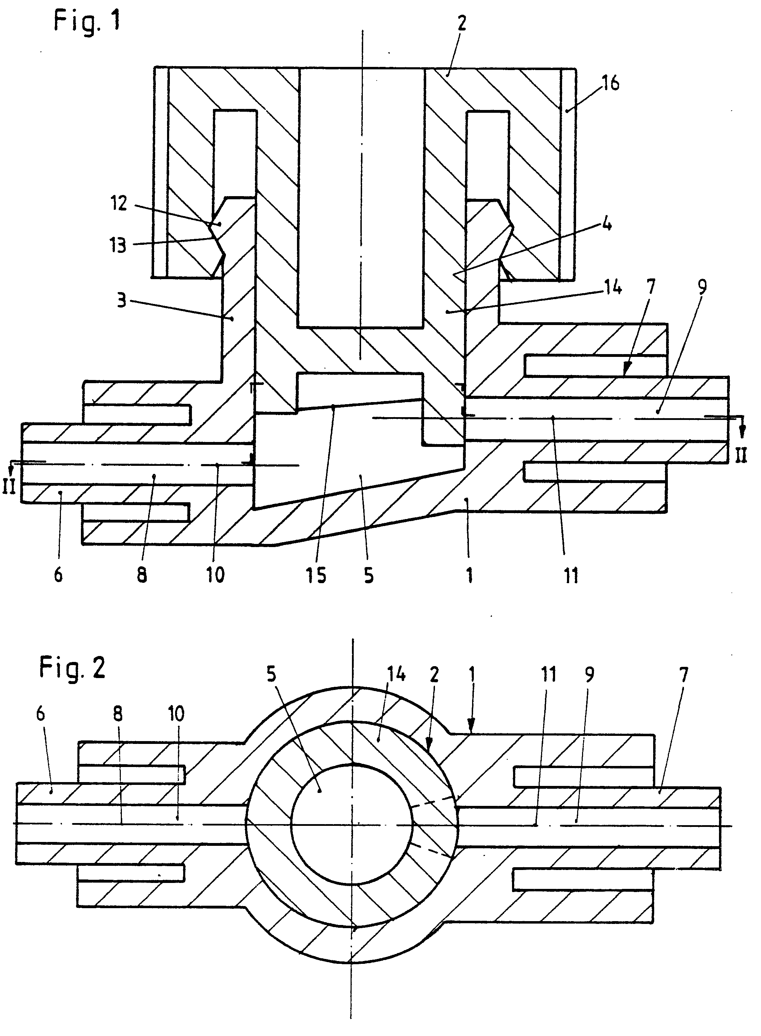

Die Dosiervorrichtung setzt sich im wesentlichen aus zwei Teilen zusammen, und zwar aus einem Hauptkörper 1 und einem beweglichen Teil 2. Der Hauptkörper 1 ist nach Art eines T-Stückes ausgebildet und besitzt innerhalb einer Wandung 3 eine Bohrung 4, die in einer Ventilkammer 5 endet. Der Hauptkörper 1 besitzt weiterhin zwei Anschlußstutzen 6 und 7, die jeweils eine Durchbrechung 8 und 9 aufweisen, die beide Anschluß an die Ventilkammer 5 besitzen. Die Durchbrechungen 8 und 9 besitzen Achsen 10 und 11, die, wie aus Fig. 1 ersichtlich ist, gegeneinander um einen gewissen Betrag versetzt angeordnet sind. Die Anschlußstutzen 6 und 7 dienen dem Anschließen von Schlauchabschnitten bzw. Schlauchleitungen, wie sie in der Medizin üblicherweise angewendet werden.

Der Hauptkörper besitzt im Endbereich seiner Wandung 3 eine umlaufende Rippe 12, der am beweglichen Teil 2 eine umlaufende Nut 13 zugeordnet ist, so daß auf diese Art und Weise der bewegliche Teil 2 unter Anwendung eines Schnappeffektes auf die Wandung 3 des Hauptkörpers 1 aufschnappbar ist, so daß er dort drehbeweglich geführt ist. Der bewegliche Teil 2 besitzt einen in die Bohrung 4 einreichenden Fortsatz 14, der gemäß Fig. 1 in einer schiefen Ebene 15 endet, die über den größten Teil des Umfanges des Fortsatzes 14 umläuft und von der in Fig. 1 etwa nur die Hälfte dargestellt ist. Jedenfalls ist der Fortsatz 14 mit seiner schiefen Ebene 15 so ausgebildet und angeordnet, daß die reine Drehbewegung des beweglichen Teils 2 dazu führt, daß die Durchbrechung 9 des Anschlußstutzens 7 bei einer Drehbewegung mehr oder weniger vollständig von der Ventilkammer 5 getrennt wird, während gleichzeitig die Durchbrechung des Anschlußstutzens 6 dauernd in Verbindung mit der Ventilkammer 5 verbleibt. Wie ersichtlich, ist durch eine Drehbewegung des beweglichen Teils 2 eine konstante Einstellung der Durchflußmenge bzw. der Tropfenzahl der jeweils durch die Dosiervorrichtung geleiteten Flüssigkeit möglich. Zur erleichterten Handhabung besitzt der bewegliche Teil 2 außen eine Rändelung, Riffelung o. dgl. 16. Es ist auch möglich, hier eine flügelartige oder eine polygone Ausbildung zu wählen. Der Fortsatz 14 ist mit einer solchen Passung in der Bohrung 4 des Hauptkörpers 1 gelagert und geführt, daß eine ausreichende Dichtung erzielt wird.

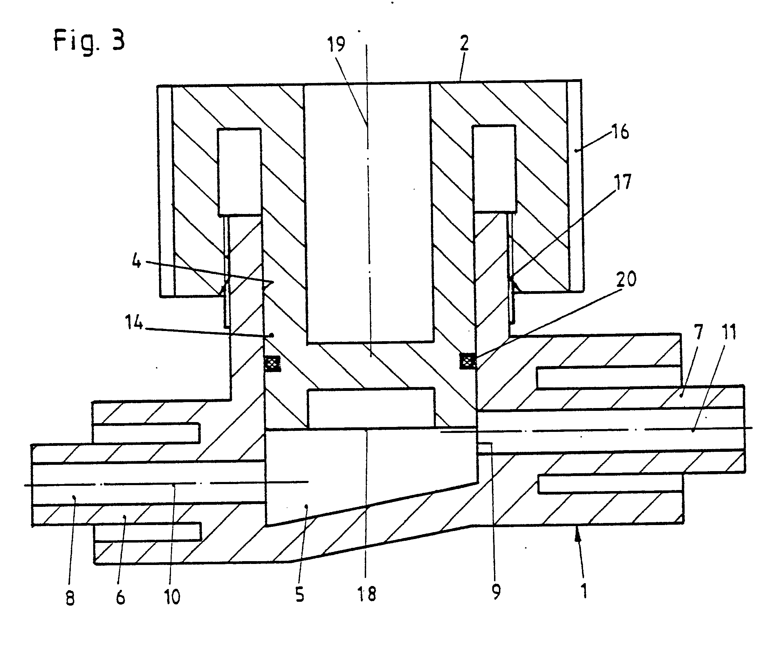

Fig. 3 zeigt eine weitere Ausführungsform. Hier ist zwischen dem beweglichen Teil 2 und dem Hauptkörper 1 eine Gewindeverbindung 17 in der dargestellten Weise vorgesehen, so daß eine Verdrehung des Teils 2 nicht nur zu einer Rotation, sondern zu einer zwangsweise zugeordneten Axialverschiebung in der Bohrung 4 des Hauptkörpers führt. Der Fortsatz 14 endet in der Bohrung 4 bzw. in der Ventilkammer 5 mit einer umlaufenden Schließkante 18, die sich somit an allen Stellen rechtwinklig zu der Achse 19 des beweglichen Teils 2 erstreckt. Zusätzlich trägt der Fortsatz 14 eine Dichtung 20. Man erkennt, wie auch hier eine besonders feinfühlige Einstellung der Durchflußmenge bzw. Tropfenzahl durch eine Verdrehbewegung des beweglichen Teils 2 gegenüber dem Hauptkörper 1 möglich ist. Hier wird auch nur die eine Durchbrechung 9 des Anschlußstutzens 7 mehr oder weniger verschlossen bzw. freigegeben.

**Patentansprüche**

1. Dosiervorrichtung zur Einstellung einer konstanten Durchflußmenge für Blut, Blutersatzlösungen und ähnliche Flüssigkeiten, mit einem Hauptkörper (1), der eine Bohrung (4) zur Aufnahme eines als Verschlußkörper dienenden beweglichen Teils (2) und zwei an die eine Ventilkammer (5) bildende Bohrung (4) rechtwinklig anschließende Anschlußstutzen (6, 7) mit Durchbrechungen (8, 9) für eine Durchflußleitung aufweist, wobei die Achsen (10, 11) der Anschlußstutzen (6, 7) bzw. Durchbrechungen (8, 9) derart versetzt zueinander im Hauptkörper (1) angeordnet sind, daß in der Ventilkammer (5) der Verschlußkörper nur im Bereich einer Durchbrechung (9) von der völligen Freigabe bis zum völligen Abschluß bewegbar gelagert ist, dadurch gekennzeichnet, daß der bewegliche Teil (2) an dem Hauptkörper (1) zum Zwecke der Ein- oder Verstellung der Durchflußmenge drehbar gelagert ist. und daß an einem in die Bohrung (4) einragenden Fortsatz (14) des beweglichen Teils (2) stirnseitig eine Schließkante (15, 18) vorgesehen ist, die je nach Drehung des Teils (2) die eine Durchbrechung (9) mehr oder weniger verschließt.

2. Dosiervorrichtung nach Anspruch 1, dadurch gekennzeichnet, daß der bewegliche Teil (2) an dem Hauptkörper (1) über eine umlaufende Rippe/Nut-Verbindung (12, 13) drehbar gelagert ist, und daß die Schließkante als schiefe Ebene (15) ausgebildet ist.

3. Dosiervorrichtung nach Anspruch 1, dadurch gekennzeichnet, daß der bewegliche Teil (2) an dem Hauptkörper (1) über eine Gewindeverbindung (17) geführt ist, und daß die Schließkante (18) rechtwinklig umlaufend zu der Achse (19) des beweglichen Teils (2) vorgesehen ist.

4. Dosiervorrichtung nach Anspruch 2 oder 3, dadurch gekennzeichnet, daß der Hauptkörper (1) und der bewegliche Teil (2) als Kunststoffspritzteile ausgebildet sind.

5. Dosiervorrichtung nach Anspruch 2 oder 3, dadurch gekennzeichnet, daß zwischen dem beweglichen Teil (2) und dem Hauptkörper (1) eine Dichtung (20) angeordnet ist.

**Claims**

1. Metering device for the setting of a constant throughflow quantity for blood, blood substitute solutions and similar liquids, with a main body (1), which displays a bore (4) for the reception of a movable body (2) serving as closure body and two connecting stubs (6, 7), which at right angles adjoin the bore (4) forming a valve chamber (5), with passages (8, 9) for a throughflow duct, wherein the axes (10, 11) of the connecting stubs (6, 7) or passages (8, 9) are arranged each displaced relative to the other in the main body (1) in such a manner that the closure body is borne in the valve chamber (5) to be movable only in the region of one passage (9) from the complete freeing to the complete closure, characterised thereby, that the movable part (2) is rotatably borne at the main body (1) for the purpose of the setting or resetting of the throughflow quantity and that a closing edge (15, 18), which in accordance with the rotation of the movable part (2) closes the one passage (9) more or less, is provided at the end face of a prolongation (14). which projects into the bore (4), of the movable part (2).

2. Metering device according to claim 1,

characterised thereby, that the movable part (2) is rotatably borne at the main body (1) by way of an encircling rib-groove connection (12, 13) and that the closing edge is formed as inclined plane (15).

3. Metering device according to claim 1, characterised thereby, that the movable part (2) is guided at the main body (1) by way of a threaded connection and that the closing edge is provided to be encircling at right angles to the axis (19) of the movable part (2).

4. Metering device according to claim 2 or 3, characterised thereby, that the main body (1) and the movable part (2) are constructed as injection-moulded synthetic material parts.

5. Metering device according to claim 2 or 3, characterised thereby, that a seal (20) is arranged between the movable part (2) and the main body (1).

**Revendications**

1. Dispositif de dosage pour la régulation d'un débit constant du sang, solutions succédanées du sang et fluides analogues, constitué d'un corps principal (1), qui présente un alésage (4) pour recevoir une pièce mobile (2) servant de corps de fermeture, et deux tubulures (6, 7) de raccordement, reliées à angle droit à l'alésage (4) formant une chambre de valve (5), et qui comportent des passages (8, 9) pour former une conduite de circulation, les axes (10, 11) des tubulures (6, 7), c'est-à-dire des passages (8, 9), étant disposés dans le corps principal avec un décalage entre eux tel que, dans la chambre de valve, le corps de fermeture est monté de façon à pouvoir se déplacer seulement dans la zone du passage (9) d'une position d'ouverture totale à une position de fermeture totale, caractérisé par le fait que la pièce mobile (2) est montée sur le corps principal (1) grâce à une combinaison nervure-gorge circulaire de façon à pouvoir tourner dans le but de réguler le débit ou de changer la régulation du débit, et qu'un bord de fermeture (15, 18) est prévu à l'extrémité d'un prolongement (14) de la pièce mobile (2) dépassant dans l'alésage (4), lequel bord de fermeture, après rotation de la pièce (2), ferme plus ou moins l'un (9) des passages.

2. Dispositif de dosage selon la revendication 1, caractérisé par le fait que la pièce mobile (2) est montée sur le corps principal (1) grâce à une combinaison nervure-gorge (12, 13) circulaire de façon à pouvoir tourner et que le bord de fermeture est conformé en un plan oblique (15).

3. Dispositif de dosage selon la revendication 1, caractérisé par le fait que la pièce mobile (2) est montée sur le corps principal (1) par un assemblage (17) à vis et que le bord (18) de fermeture est prévu à angle droit sur tout le tour par rapport à l'axe (19) de la pièce mobile (2).

4. Dispositif de dosage selon la revendication 2 ou 3, caractérisé par le fait que le corps principal (1) et la pièce mobile (2) sont réalisés par injection.

5. Dispositif de dosage selon la revendication 2 ou 3, caractérisé par le fait qu'entre la pièce mobile (2) et le corps principal (1) on dispose un joint (20).

Fig. 1

Fig. 2

Fig. 3